# EUROPÄISCHE PATENTSCHRIFT

(11) **EP 1 206 485 B1**
(45) Veröffentlichungstag und Bekanntmachung des Hinweises auf die Patenterteilung: **21.03.2007**
(21) Anmeldenummer: 00953186.4
(22) Anmeldetag: 20.08.2000
(51) Int. Cl.: C07K 5/037, C07K 7/02, C07D 277/04, A61K 31/425, A61K 38/06, A61P 3/00

(54) **NEUE EFFEKTOREN DER DIPEPTIDYL PEPTIDASE IV ZUR TOPISCHEN ANWENDUNG**
NEW EFFECTORS OF DIPEPTIDYL PEPTIDASE IV FOR TOPICAL USE
NOUVEAUX EFFECTEURS DE DIPEPTIDYLPEPTIDASE IV DESTINES A UNE APPLICATION TOPIQUE

(30) Priorität: 24.08.1999 DE 19940130
(43) Veröffentlichungstag der Anmeldung: 22.05.2002
(73) Patentinhaber: Probiodrug AG, 06120 Halle/Saale (DE)
(72) Erfinder: DEMUTH, Hans-Ulrich, 06120 Halle (DE); HOFFMANN, Torsten, 06120 Halle (DE); SCHLENZIG, Dagmar, 06114 Halle (DE); HEISER, Ulrich, 06120 Halle (DE)
(74) Vertreter: Hoffmann, Matthias
(86) Internationale Anmeldenummer: PCT/EP2000/008118
(87) Internationale Veröffentlichungsnummer: WO 2001/014318

(56) Entgegenhaltungen:
- WO-A-95/15309
- WO-A-95/34538
- DE-A- 19 826 972

## Beschreibung

Die Erfindung betrifft neue Effektoren der Dipeptidyl Peptidase IV. Diese Effektoren können zur gezielten Beeinflussung lokal begrenzter pathophysiologischer und physiologischer Prozesse (Entzündungen, Chemotaxis, Autoimmunerkrankungen, Wundheilung) eingesetzt werden. Dabei werden die enzymatische Aktivität und die Bindungsaktivitäten der Dipeptidyl Peptidase IV und von Enzymen mit vergleichbarer oder identischer Aktivität sowie von Proteinen mit verwandter Primärstruktur (z. B. FAP, Fibroplast Activation Protein (Levy et al., 1999)) mittels Effektoren (Substrate, Pseudosubstrate, Inhibitoren, Antikörpern, Bindungsproteine, Bindungsantagonisten, Bindungsagonisten u.a.) beeinflußt.

Neben Proteasen, die in unspezifische Proteolyse einbezogen sind, was letztlich den Abbau von Proteinen zu Aminosäuren bewirkt, kennt man regulatorische Proteasen, die an der Funktionalisierung (Aktivierung, Deaktivierung, Modulierung) von endogenen Peptidwirkstoffen beteiligt sind (Kirschke *et al.,* 1995) Kräusslich and Wimmer, 1987). Insbesondere im Zusammenhang mit der Immunforschung und der Neuropeptidforschung sind eine Reihe solcher sogenannten Konvertasen, Signalpeptidasen oder Enkephalinasen entdeckt worden (Gomez *et al.,* 1988)(Ansorge *et al.,* 1991).

Aufgrund der Häufigkeit des Vorkommens der Aminosäure Prolin in einer Vielzahl von Peptidhormonen und den damit verbundenen Struktureigenschaften dieser Peptide wird für prolinspezifische Peptidasen eine den Signalpeptidasen analoge Funktion diskutiert (Yaron and Naider, 1993); (Vanhoof *et al.,* 1995). Dabei bestimmt Prolin in diesen Peptiden durch seine besondere Struktur sowohl Konformation als auch Stabilität dieser Peptide, indem es sie vor Abbau durch unspezifische Proteasen schützt (Kessler, 1982). Enzyme, die dagegen hochspezifisch strukturverändernd auf Prolin-haltige Sequenzen einwirken (HIV-Protease, Cyclophylin u. a.) sind attraktive Ziele der aktuellen Wirkstoff-Forschung. Insbesondere für die nach dem Prolin spaltenden Peptidasen Prolyl Endopeptidase (PEP) und Dipeptidyl Peptidase IV (DP IV) konnten Beziehungen zwischen der Modulation der biologischen Aktivität von natürlichen Peptidsubstraten und deren selektiver Spaltung durch diese Enzyme wahrscheinlich gemacht werden. So nimmt man an, daß PEP eine Rolle beim Lernen bzw. im Gedächtnisprozeß spielt und DP IV in die Signalübertragung während der Immunantwort einbezogen ist (Ishiura *et al.,* 1989); (Hegen *et al.,* 1990).

DP IV- bzw. DP IV-analoge Aktivität (z. B. besitzt die lysosomale DP II eine der DP IV nahezu identische Substratspezifität) kommt im Blutkreislauf sowie in nahezu allen Organen vor, wo sie hochspezifisch Dipeptide vom N-Terminus biologisch aktiver Peptide abspaltet, wenn Prolin oder Alanin die benachbarten Reste der N-terminalen Aminosäure in deren Sequenz darstellen. Deshalb wird davon ausgegangen, daß dieses Enzym an der Regulation der biologischen Aktivität von Polypeptiden *in vivo* beteiligt ist (Vanhoof *et al.,* 1995).

Kürzlich wurde gezeigt, daß eine Reihe von Chemokinen (RANTES, SDF-1alpha, MDC, Eotaxin u.a.) Substrate der DP IV sind und diese durch DP IV in ihrer Funktion moduliert werden (Proost *et al.,*1998; Proost *et al.,* 1998; Proost *et al., 1999);* (Shioda *et al.,* 1998)). Chemökine sind durch ihre chemotaktische Wirkung wesentlich an der Regulation lokaler immunologischer Vorgänge, wie Autoimmunprozesse, Enzündungen und Wundheilung beteiligt (Nelson and Krensky, 1998). In neueren Arbeiten konnte von uns nachgewiesen werden, daß auch biologisch aktive Peptide mit Serin oder Threonin in P₁-Position (Glukagon, VIP, PACAP) Substrate der DP IV darstellen.

Eine Reihe von biologisch aktiven DP IV-Substraten (Substanz P, Somatostatin, VIP, PACAP u.a.) sind an der Regulation von neuronalen, immunologischen und vasoaktiven Prozessen in der Haut beteiligt (Scholzen *et al., 1998);* (Wallengren, 1997). Die Dipeptidyl Peptidase IV stellt somit eine wichtige Schaltstelle bei der Regulation der Aktivität gastrointestinal, immunologisch bzw. neurologisch aktiver Peptide und damit ein interessantes therapeutisches Target dar (Augustyns *et al.,* 1999). Die genauen Signalkaskaden sind im einzelnen jedoch nicht vollständig aufgeklärt.

Genauere Kenntnisse existieren über die Rolle der DP IV bei der Regulation der Blutzuckers. Durch limitierte Proteolyse werden die Inkretine GIP₁₋₄ und GLP-1₇₋₃₇ inaktiviert. Eine Hemmung der Plasma-DP IV-Aktivität führt über eine verlängerte Aktivität der Inkretine und eine verstärkte Insulinfreisetzung zu einer Normalisierung des Blutzuckerspiegels (Demuth *et al.,* 1996; Pauly *et al.,* 1999; Pauly *et al.,* 1996).

Die Rolle die DP IV im Immunsystem ist noch nicht vollständig geklärt. Sie ist ein Aktivierungsmarker von T-Lymphozyten sowie ein Rezeptor für die Adenosindeaminase. Der Einsatz von DP IV-Inhibitoren hat in der Zellkultur und *in vivo* immunsuppressive Effekte (Ansorge *et al.,* 1995; Reinhold *et al.,* 1997; Kubota *et al.,* 1992). Mit monoklonalen Antikörpern gegen CD26 wurden zum Teil unabhängig von der enzymatischen Aktivität des Enzyms stimulatorische Effekte auf intrazelluläre Signalkaskaden (Ca²⁺-influx, Kinaseaktivierungen) erzielt (Hegen *et al.,* 1993; *Kameoka et* a/., 1995; Tanaka *et al.,* 1993; Kähne *et al.,* 1995).

Aus der N-terminalen Sequenz der Substanz P abgeleitete Lysyl-Prolyl-Analoga zeigten einen fördernden Effekt auf die Wundheilung, der auf die Strukturähnlichkeit zu Substanz P zurückgeführt wird. Systemisch eingesetzte irreversible DP IV-Inhibitoren führten dagegen zu einer Hemmung der Wundheilung (Buntrock *et al.,* 1988; Kohl *et al.,* 1991; Kohl *et al.,* 1989).

Neben der Anwendung von DP IV- Inhibitoren zur Normalisierung der Blutglukose wurden DP IV-Inhibitoren bisher systemisch für die Behandlung von Arthritis im Tiermodell eingesetzt. Bei Arthritis-Patienten sowie in tierischen Arthritismodellen wurde eine Verringerung der DP IV-Aktivität beobachtet (Küllertz and Boigk, 1986) (Fujita *et al.,* 1992). Insbesondere durch orale oder subkutane Applikation von systemisch wirkenden DP IV Inhibitoren wurde im Tiermodell eine Unterdrückung der Alkyldiamin-induzierten Arthritis erreicht (Tanaka *et al.,* 1997; Tanaka *et al.,* 1998).

Auch im Zusammenhang mit anderen Autoimmunerkrankungen wurde eine Wirkung durch DP IV-Inhibitoren erzielt. So konnte durch DP IV-Inhibierung eine Unterdrückung der Proliferation von ,mylin basic protein'-spezifischen T-Zell-Klonen erreicht werden (Reinhold *et al.,* 1998). Bei verschiedenen Hautkrankheiten (Psoriasis, Lichen planus) und kanzerogenen Erkrankungen der Haut konnte eine erhöhte DP IV-Aktivität in Keratinozyten und Fibroblasten nachgewiesen werden (Novelli *et al., 1996)* (Raynaud *et al., 1992).* Auch das eng mit DP IV verwandte ,fibroblast-activation protein`, das ca. 50% Sequenzhomologie zur DP IV aufweist und wahrscheinlich mit der durch Piñeiro-Sanchez *et al*., 1997 beschriebenen Seprase identisch ist, wird verstärkt von inaktivierten Fibroblasten von Epithelkarzinomen und heilenden Wunden exprimiert (Niedermeyer *et al.,* 1998).

Auf Grund der weiten Verbreitung des Proteins im Organismus und der vielfältigen Mechanismen in die DP IV, DP IV-Aktivitäten und DP IV-verwandte Proteine involviert sind, kann eine systemische Therapie (enterale oder parenterale Applikation) mit DP IV-Inhibitoren zu einer Reihe unerwünschter Nebenwirkungen führen. So greift eine parenterale oder enterale Applikation von DP IV-Inhibitoren regulierend bzw. deregulierend in den Glukosestoffwechsel ein.

Es konnte nun gezeigt werden, daß Seitenketten-modifizierte Substrate des Enzyms Dipeptidyl Peptidase IV durch das Enzym erkannt und wie unmodifizierte Substrate gespalten werden können.(DEMUTH, H.-U., HEINS, J., 1995).

So konnte z.B. gezeigt werden, daß phosphorylierte Dipeptid-(B)-*p*-nitroanilide [KASPARI, A., et al., 1996] Substrate der DP IV sind. DP IV-Inhibitoren wie z.B. Glu(Gly)-Thia oder Lys(Z-NO₂)-Thia [REINHOLD, D., et al., 1998] werden vollständig transportiert.

Die zu lösende Aufgabe bestand darin, Verbindungen herzustellen, die zur gezielten Beeinflussung lokal begrenzter pathophysiologischer und physiologischer Prozesse eingesetzt werden können. Insbesondere besteht die Aufgabe der Erfindung darin, eine lokal begrenzte Hemmung von DP IV bzw. DP IV-analoger Aktivität zu erzielen, um damit gezielt in die Regulation der Aktivität von lokal wirksamen Peptidhormonen eingreifen zu können.

Diese Aufgabe wird erfindungsgemäß durch die Bereitstellung von Verbindungen der allgemeinen Formeln 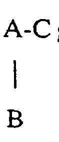 gelöst, wobei
A eine Aminosäure mit mindestens einer funktionellen Gruppe in der Seitenkette ist,
B eine chemische Verbindung ist, die kovalent an mindestens eine funktionelle Gruppe der Seitenkette von A gebunden ist, nämlich
- Oligopeptide mit einer Kettenlänge von bis zu 20 Aminosäuren, außer Homopolymeren von Glycin aus bis zu 6 Glycinmonomeren, oder
- Polyethylenglykole mit Molmassen von bis zu 20000 g/mol, und
C eine Thiazolidin-, Pyrrolidin-, Cyanopyrrolidin, Hydroxyprolin-, Dehydroprolin- oder Piperidingruppe ist, die in Amidbindung mit A vorliegt.

Insbesondere wird erfindungsgemäß mindestens eine pharmazeutische Zusammensetzung bereitgestellt, die
mindestens eine Verbindung der allgemeinen Formeln 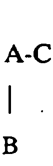 enthält.

Vorzugsweise ist A eine α-Aminosäure, besonders bevorzugt eine natürliche α-Aminosäure vorzugsweise Threonin, Tyrosin, Serin, Arginin, Lysin, Asparaginsäure, Glutaminsäure oder Cystein.

Vorzugsweise sind die Oligopeptide Homopolymere, Copolymere oder Blockcopolymere.

Ferner werden derartige Verbindungen bzw. pharmazeutische Zusammensetzungen zur topischen Beeinflussung insbesondere der Verringerung der Aktivität von Dipeptidyl-Peptidase IV oder analoger Enzyme verwendet.

In der gesamten Beschreibung und den Ansprüchen kann der Ausdruck "Alkyl" eine C₁₋₅₀ Alkylgruppe, bevorzugt eine C₆₋₃₀ Alkylgruppe, vorzugsweise eine C₈₋₁₂ Alkylgruppe darstellen; so kann eine Alkylgruppe z.B. eine Methyl-, Ethyl-, Propyl-, Isopropyl- oder Butylgruppe sein;
ist der Ausdruck "Alk" z.B. in dem Ausdruck "Alkoxy" und der Ausdruck "Alkan" z.B. in dem Ausdruck "Alkanoyl" wie "Alkyl" definiert;
sind Aromaten bevorzugt substituierte oder gegebenenfalls unsubstituierte Phenyl-, Benzyl-, Naphthyl-, Biphenyl- oder Anthracengruppen, die vorzugsweise mindestens 8 C-Atome aufweisen;
kann der Ausdruck "Alkenyl" eine C₂₋₁₀ Alkenylgruppe, vorzugsweise eine C₂₋₆ Alkenylgruppe darstellen, die die Doppelbindung(en) an beliebiger Stelle aufweist und unsubstituiert oder substituiert sein kann;
kann der Ausdruck "Alkinyl" eine C₂₋₁₀ Alkinylgruppe, vorzugsweise eine C₂₋₆ Alkinylgruppe darstellen, die die Dreifachbindung(en) an beliebiger Stelle aufweist und unsubstituiert oder substituiert sein kann;
kann der Ausdruck "substituiert" oder Substituent eine beliebige Substitution durch eine oder mehrere, vorzugsweise eine oder zwei Alkyl-, Alkenyl-, Alkinyl-, ein- oder mehrwertige Acyl-, Alkanoyl-, Alkoxyalkanoyl- oder Alkoxyalkylgruppen darstellen; die vorstehenden Substituenten können wiederum eine oder mehrere, vorzugsweise jedoch keine, Alkyl-, Alkenyl-, Alkinyl-, ein- oder mehrwertige Acyl-, Alkanoyl-, Alkoxyalkanoyl- oder Alkoxyalkylgruppen als Seitengruppen aufweisen;
können organische Amine, Amide, Alkohole oder Säuren mit jeweils 8 bis 50 C-Atomen vorzugsweise mit 10 bis 20 C-Atomen die Formeln (Alkyl)₂N- oder Alkyl-NH-, -CO-N(Alkyl)₂ oder -CO-NH(Alkyl), -Alkyl-OH oder -Alkyl-COOH aufweisen.

Die erfindungsgemäßen Verbindungen können trotz verlängerter Seitenkeitenfunktion noch an das aktive Zentrum des Enzyms Dipeptidyl Peptidase IV und analoger Enzyme binden, werden aber nicht mehr aktiv durch den Peptidtransporter PepT1 transportiert. Die daraus resultierende verminderte bzw. stark eingeschränkte Transportfähigkeit der erfindungsgemäßen Verbindungen führt in idealer Weise zu einer lokalen, topischen Inhibierung der DP IV und analoger Enzyme.

Die erfindungsgemäßen Verbindungen bzw. erfindungsgemäß verwendeten Verbindungen können als Racemate oder als enantiomerenreine Verbindungen, vorzugsweise in der L-threo- oder L-allo-Form, bezogen auf A, vorliegen bzw. verwendet werden.

Ein lokaler Eingriff in die Regulation von Peptidhormonen durch topische Applikation von spezifischen DP IV-Inhibitoren bietet damit die Möglichkeit, durch enterale oder parenterale Gabe von DP IV-Inhibitoren verursachte systemische Nebenwirkungen zu vermeiden, da nur eine lokal begrenzte Hemmung der DP IV-Aktivität stattfindet. Systemische Regulations-prozesse bzw. Regulationsprozesse in anderen Geweben bleiben weitestgehend unbeeinflußt, da eine schnelle systemische Verteilung dieser Verbindungen vermieden wird.

Durch Verlängerung / Vergrösserung der Seitenkettenmodifikationen z.B. über eine Kohlenstoffanzahl von sieben C-Atomen hinaus, kann erfindungsgemäß also eine dramatische Reduktion der Transportierbarkeit erzielt werden (Tabelle 1). Die Beispiele in Tabelle 1 zeigen deutlich, daß mit zunehmender räumlicher Grösse der Seitenketten die Transportfähigkeit der Substanzen abnimmt. Durch die räumliche und sterische Vergrösserung der Seitenketten z.B. über die Atomgruppengrösse eines einfach substituierten Phenylrestes, Hydroxylaminrestes oder Aminosäurerestes hinaus, kann man erfindungsgemäss die Transportierbarkeit der Zielsubstanzen modulieren bzw. supprimieren.

Damit wird eine diskriminierende Beeinflussung der DP IV-Aktivität im lebenden Organismus ermöglicht.

Durch die Erfindung kann damit zum einen eine effektive Wirkung der Inhibitoren im zu behandelnden Gewebe erreicht werden, und zum anderen können durch lokal begrenzte, also topische Applikation von DP IV-Inhibitoren systemische Wirkungen der Inhibitoren weitestgehend vermieden werden. Damit wird eine effektive und nebenwirkungsarme Beeinflussung lokaler physiologischer und pathophysiologischer Prozesse (Entzündungen, Psoriasis, Arthritis, Autoimmunerkrankungen, Allergien) erreicht.

Die Erfindung stützt sich auf folgende Tatsachen:
- Enteral bzw. parenteral, d. h. oral bzw. i.v. oder subkutan verabreichte DP IV-Inhibitoren verteilen sich systemisch und hemmen die DP IV und analoge Aktivitäten im gesamten Organismus.
- Eine Reihe von bioaktiven Peptidsubstraten der DP IV sind dagegen in die Regulation lokaler Signalkaskaden involviert (Chemotaxis, Entzündungen, Neurotransmission).
- Überraschenderweise zeigen die erfindungsgemäßen seitenkettenmodifizierten DP IV-Inhibitoren eine hohe inhibitorische Potenz, werden aber kaum bzw. nicht absorbiert und transportiert und führen damit nicht zu nachweisbaren systemischen Effekten.

Die Erfindung stellt also neue DP IV-Inhibitoren und eine neuartige Herangehensweise für die Anwendung der DP IV- Inhibitoren *in vivo* bereit. Entsprechende Inhibitoren lassen sich durch chemische Modifizierungen bzw. Formulierungen auf die Art der Anwendung anpassen. So wird z.B. durch voluminöse hydrophile Substitutionen an der Seitenkette eine systemische Verteilung erschwert bzw. vermieden.

Die Inhibitoren können in pharmazeutischen und kosmetischen Präparaten verabreicht werden.

Die topische Anwendung umfaßt dabei die lokale Anwendung der Inhibitoren durch direktes Aufbringen auf das zu behandelnde Gewebe (z.B. Haut, Wunden, Tumore) durch Salben, Cremes, Kosmetika, oder indirektes Aufbringen durch effektorhaltige Pflaster, Binden o. ä., durch Applikation in Körperteile (Mund, Nase, Ohren, Augen, Lunge) in Form von Tropfen, Sprays, Inhalaten o. ä., durch direkte Injektion in oder um das zu behandelnde Gewebe und durch Implantation von effektorhaltigen Materialien. Die topische Anwendung umfaßt weiterhin die orale oder anale Applikation schwer- bzw. nichtabsorbierbarer Effektoren der Dipeptidyl Peptidase IV bzw. DP IV-analoger Sequenzen zur selektiven Beeinflussung der gastro-intestinalen DP IV.

Erfindungsgemäß werden insbesondere Verbindungen eingesetzt, in denen die Oligopeptide Kettenlängen von 3 bis 15, besonders bevorzugt von 4 bis 10 Aminosäuren aufweisen, und/oder die Polyethylenglykole mit Molmassen von mindestens 250 g/mol, vorzugsweise von mindestens 1500 g/mol und bis zu 15000 g/mol aufweisen, und/oder die gegebenenfalls substituierten organischen Amine, Amide, Alkohole, Säuren oder Aromaten mit mindestens 12 C-Atomen und vorzugsweise bis zu 30 C-Atomen aufweisen. Weiter werden pharmazeutische und kosmetische Zusammensetzungen offenbart, die mindestens eine erfindungsgemäße Verbindung gegebenenfalls in Kombination mit an sich üblichen Trägern oder Adjuvantien enthält.

Die erfindungsgemäßen Verbindungen oder pharmazeutischen oder kosmetischen Zusammensetzungen können zur topischen Beeinflussung der Aktivität von Dipeptidylpeptidase IV oder analoger Enzyme, insbesondere zur Prophylaxe oder Therapie von Haut- oder Schleimhauterkrankungen, Autoimmunerkrankungen und Entzündungen wie z.B. Psoriasis, Allergien, Arthritis, Tumoren oder Autoimmunerkrankungen eingesetzt werden.

DieVerbindungen und pharmazeutischen oder kosmetischen Zusammensetzungen können als Salbe, Creme, Kosmetikum, Pflaster, Binde, Tropfen, Spray, Inhalat, Implantat oder Injektionslösung formuliert und eingesetzt werden.

Die erfindungsgemäß eingesetzten Adjuvantien sind an sich bekannt.

Die Erfindung betrifft also die topische Anwendung von Effektoren der Dipeptidyl Peptidase IV sowie DP IV-analoger Enzymaktivitäten und DP IV-ähnlicher Proteine. Die topische Anwendung erlaubt eine lokale Modulation der Aktivitäten der obengenannten hochspezifischen Enzyme, die entscheidend an der Inaktivierung und Aktivierung biologisch aktiver Peptide (Chemokine, Substanz P, VIP, PHM, PACAP, Wachstumsfaktoren, u.a.) beteiligt sind.
Damit ist ein gezieltes Eingreifen in lokale immunologische Prozesse (Chemotaxis, Entzündungsprozesse, Autoimmunerkrankungen) und eine wirksame und gezielte Behandlung damit verbundener pathophysiologischer und physiologischer Prozesse (Psoriasis, Parodontitis, Arthritis, Allergien, Entzündungen) möglich. Die Erfindung ermöglicht eine leichte Anwendbarkeit der Inhibitoren in hohen lokalen Konzentrationen.

Durch die geringe systemische Belastung mit den entsprechenden Effektoren wird eine Beeinflussung des Inkretinsystems sowie der systemischen Immunantwort vermieden.

### Ausführungsbeispiele

### Beispiel 1: Wirkung von seitenkettenmodifizierten Glutamylthiazolidinen als schwer transportierbaren DP IV Inhibitoren

Es wurden seitenkettenmodifizierte Glutamylthiazolidine der Struktur H-Glu(X)-Thia synthetisiert, wobei als X Polyethylenglycol oder Glycinoligomere verschiedener Kettenlänge verwendet wurden (Synthesebeschreibung siehe Methode A). Von diesen Derivaten wurden die Bindungseigenschaften am und die Transportfähigkeit durch den Peptidtransporter PepT1 untersucht sowie die Kᵢ-Werte gegenüber DP IV bestimmt (Tabelle 1).

Überraschenderweise konnte festgestellt werden, daß die Seitenkettenmodifikationen die Bindungs-Eigenschaften an der Verbindung nur in geringem Maße verändern. Dagegen wird die Transportfähigkeit der Inhibitoren durch den Peptidtransporter durch die Seitenkettenmodifizierung drastisch herabgesetzt.

Damit eignen sich diese DP IV-Inhibitoren hervorragend, um eine lokal begrenzte (topische) Hemmung der DP IV im Organismus zu erreichen.

**Tabelle 1: Transportierbarkeit und Inhibitorkonstanten von ausgewählten DP IV-Inhibitoren.**

| **Verbindung** *Aminosäurethiazolidide* | **EC50 (mM)¹** | **Iₘₐₓ (nA)²** | **Kᵢ (Mol/l)³** |
|---|---|---|---|
| **H-Ile-Thia** | 0,98 | 25 ± 8 | 1,3e-7 ± 11,1% |
| **H-Glu-Thia** | 1,1 | 35±13 | 6,1e-7±11,4% |
| *Seitenkettenmodifizierte Glutanzylthiazolidine* | | | |
| **H-Glu(NHOH)-Thia** | 3,18 | 42±11 | 1,7e-6±8,6% |
| **H-Glu(Gly₃)-Thia** | 8,54 | n.n.⁴ | 1,92e-7± 8,4% |
| **H-Glu(Gly₅)-Thia** | >10 | n.n.⁴ | 9,93e-8±11,4% |
| **H-Glu(PEG)-Thia** | >10 | n.n.⁴ | 3,11e-6±9,8% |

| | | | |
|---|---|---|---|
| ¹ Effektive Konzentrationen der Verbindungen, die die Bindung von ³H-D-Phe-Ala (80mM) an PepT1-exprimierende *P. pastoris-Zellen* zu 50% inhibieren (EC₅₀-Werte) ² Transponverhalten an PepT1-exprimierenden Oocyten von X. leavis - mittels Zwei-Elektroden-Spannungsclamp-Methode, I = durch den Transport generierte Einwärtsströme ³ Inhibitorkonstanten für die kompetitive Hemmung von gereinigter Nieren-DP IV durch die Beispielverbindungen ⁴ nicht nachweisbar | | | |

### Beispiel 2: Effekt von oral applizierten DP IV-Inhibitoren auf Aktivität der Serum-DPIV

Die Hemmung der Plasma DP IV (systemische Wirkung) wurde nach oraler Applikation von seitenkettenmodifizierten DP IV Inhibitoren (5µM/300mg Ratte) im Vergleich zu unmodifizierten Inhibitoren an gesunden Wistar-Ratten untersucht.

Obwohl die Inhibitoren annähernd gleiche Kᵢ-Werte gegenüber DP IV besitzen (Tabelle 1) wird die Plasma-DP IV durch die neuartigen seitenkettenmodifizierten Inhibitoren deutlich langsamer und insgesamt wesentlich weniger gehemmt. Das heißt, daß diese Inhibitoren deutlich schlechter oder gar nicht aus dem Darm absorbiert werden. Insbesondere im Falle des Glu(Gly)₅-Thia ist keine systemische Wirkung des oral applizierten Wirkstoffes nachweisbar.

Damit können diese Inhibitoren als Leitstruktur für die Synthese neuartiger topisch applizierbarer DP IV-Inhibitoren ohne systemische Wirkung fungieren.

### Beispiel 3: Synthese von seitenkettenmodifizierten Inhibitoren der DP IV

### 3.1 Synthese des Boc-Glu-Thia

Umsetzung von Boc-Glu(OMe)-OH mit Thia*HCl nach Methode B (Methoden siehe Abschnitt 3.4), Verseifung des Boc-Glu(OMe)-Thia nach Methode G

**Tabelle 2 Analytische Daten des Boc-Glu-Thia**

| Verbindung | Summenformel Mᵣ Synthesemethode Ausbeute | MS [M+H]⁺ DC:R_{f}/System Fₚ | [α]²⁰D. Konzentration LM | Elementaranalyse (ber./gef.) % | HPLC Rₜ [min]/ System |
|---|---|---|---|---|---|
| Boc-Glu-Thia | C₁₃H₂₂N₂O₅S 318,38 B + G 62% | 319,5 0,52 / A¹ 0,42 /B¹ 115-118°C | -3,1 c = 1 Methanol | C:49,04/48,89 H:6,96/6,82 N:8,80/8,59 | 13,93 / A² |

| | | | | | |
|---|---|---|---|---|---|
| ¹ Dünnschichtchromatographie System A: Chloroform/Methanol 90:10 System B: Benzen/Aceton/Essigsäure 25:10:0,5 System C: n-Butanol/EE/Essigsäure/H₂O 1:1:1:1 ² HPLC-Trennbedingungen Säule: Nucleosil C-18, 7µ, 250mm x 21mm Eluent: Isokratisch, 40% ACN/Wasser/0,1% TFA Fluß: 6ml/min λ = 220nm | | | | | |

### 3.2 Seitenkettenmodifizierte Boc-Glutamylthiazolidine

Boc-Glu-Thia wurde an der γ-Carbonsäurefunktion durch die Einführung verschieden großer Reste modifiziert. Diese Reste wurden über ihre Aminogruppe durch Ausbildung einer Amidbindung an die γ-Carbonsäurefunktion gekuppelt. Dabei kamen je nach Rest verschiedene Kupplungsverfahren zum Einsatz.

Folgende Aminokomponenten wurden mit der angegebenen Methode an Boc-Glu-Thia geknüpft:

| Aminokomponente | Kupplungsmethoden (siehe Abschnitt 3.4) | Ausbeuten |
|---|---|---|
| Polyethylenglycolamin (Mᵣ ≈ 8000) | C | 93% |
| H-Gly-Gly-Gly-OH | D + E | 49% |
| H-Gly-Gly-Gly-Gly-Gly-OH | D + E | 86% |

Die Aufreinigung der Reaktionsprodukte weicht in 2 Fällen von der allgemeinen Synthesebeschreibung ab.

### Boc-Glu(Gly₅)-Thia

Das Produkt fällt bereits im Ansatz beim Rühren über Nacht aus, wird anschließend abfiltriert und mit 0,1n HCl und reichlich Wasser gewaschen. Danach wird es über P₄O₁₀ i. Vak. getrocknet.

### Boc-Glu(PEG)-Thia

Abweichend von der allgemeinen Vorschrift werden die Ausgangsstoffe der Synthese in einem 500fachen Überschuß an DMF gelöst. Nach Reaktionsende wird das DMF i.Vak. vollständig entfernt und der Rückstand in viel Methanol gelöst. Nach Überschichtung mit Ether fällt das Produkt zusammen mit nicht umgesetztem PEG aus. Die Feinreinigung erfolgte durch präparative HPLC-Trennung an einer Gelfiltrationssäule (Pharmazia, Sephadex G-25, 90µm, 260mm-100mm).
Trennbedingungen: Eluent: Wasser, Fluß: 5ml/min, λ = 220nm

**Tabelle 3 Synthesedaten der seitenkettenmodifizierten Boc-Glutamylthiazolidine**

| Verbindung | Summenformel Mᵣ Ausbeute | MS [M+H]⁺ DC/R_{f}/System Fₚ | [α]²⁰D, Konzentration LM | Elementarana lyse (ber./gef.) % | HPLC Rₜ [min]/ System |
|---|---|---|---|---|---|
| Boc-Glu(Gly₃)-Thia | C₁₉H₃₁N₅O₈S 489,54 49% | 490,5 | | C:46,62 H:6,38 N:14,31 | |
| Boc-Glu(Gly₅)-Thia | C₂₃H₃₇N₇O₁₀S 603,64 86% | 604,5 0,09 / C Zers. ab 202°C | n. b. | C:45,76/5,60 H:6,18/6,11 N:16,24/16,5 6 | 11,93 / A² |
| Boc-Glu(PEG)-Thia | 93% | ≈8000 (Massenschwepunkt) 52-53°C | n. b. | n.b. | n.b. |

| | | | | | |
|---|---|---|---|---|---|
| ² HPLC-Trennbedingungen Säule: Nucleosil C-18, 7µ, 250mm x 21mm Eluent: Isokratisch, 40% ACN/Wasser/0,1% TFA Fluß: 6ml/min λ = 220nm . | | | | | |

### 3.3 Seitenkettenmodifizierte Glutamylthiazolidine

Von den in Tabelle 3 beschriebenen Verbindungen wurden nach Methode F die N-terminalen Boc-Schutzgruppen abgespalten. Die mit Gly-Derivaten modifizierten Substanzen wurden durch präparative HPLC-Trennung aufgereinigt, und liegen als Trifluoracetate vor. Das H-Glu(PEG)-Thia wurde wie die Boc-geschützte Vorstufe an einer Gelfiltrationssäule gereinigt.

**Tabelle 4 Synthesedaten der seitenkettenmodifizierten Glutamylthiazolidine**

| Verbindung | Summenformel Mᵣ Ausbeute | MS [M+H]⁺ DC/R_{f}/System Fₚ | [α]²⁰D, Konzentration LM | Elementarana lyse (ber./gef.) % | HPLC Rₜ [min]/ System |
|---|---|---|---|---|---|
| H-Glu(Gly₃)-Thia *TFA | C₁₆H₂₄N₅O₈SF₃ 503,45 94% | 503,45 0,32 / C 91-94°C | +4,1 c = 1 Methanol | C:38,17/37,56 H:4,80/4,78 N:13,91/13,4 3 | 7,84/C° |
| H-Glu(Gly₅)-Thia *TFA | C₂₀ H₃₀N₇O₁₀SF 3 617,55 98% | 617,55 0,25 / C 105-107°C | n. b. | C:38,90/38,82 H:4,90/4,79 N:15,88/15,3 9 | 8,22 / C³ |
| H-Glu(PEG)-Thia *HCl | 92% | ≈8000 (Massenschwerpunkt) | n. b. | n. b. | n. b. |

| | | | | | |
|---|---|---|---|---|---|
| ³ HPLC-Trennbedingungen Säule: Nucleosil C-18, 7µ, 250mm x 21mm Eluent: ACN/Wasser/0,1 % TFA Gradient: 20% ACN → 90% ACN in 30min Fluß: 6ml/min λ = 220nm n.b. - nicht bestimmt *oder* nicht bestimmbar | | | | | |

### 3.4 Allgemeine Synthesevorschriften

### Methode A: Peptidbindungsknüpfung mittels Mischanhydridmethode mit CAIBE als Aktivierungsreagenz

10mmol N-terminal geschützte Aminosäure bzw. Peptid werden in 20ml absolutem THF gelöst. Die Lösung wird auf -15 °C ± 2°C abgekühlt. Nacheinander werden unter Rühren je 10mmol N-MM und 10mmol Chlorameisensäureisobutylester zugegeben, wobei streng auf die Einhaltung des angegebenen Temperaturbereichs geachtet wird. Nach ca 6min werden 10mmol der Aminokomponente zugesetzt. Handelt es sich bei der Aminokomponente um ein Salz, wird der Reaktionsansatz anschließend mit weiteren 10mmol N-MM versetzt. Danach wird die Reaktionsmischung 2h in der Kälte und über Nacht bei RT gerührt.

Der Reaktionsansatz wird einrotiert, in EE aufgenommen, mit mit 5%iger KH₂SO₄-Lösung, gesättigter NaHCO₃-Lösuna und gesättigter NaCl-Lösung gewaschen und über NaSO₄ getrocknet. Nach der Entfernung des LM i. Vak. wird die Verbindung aus EE/Pentan umkristallisiert.

### Methode B: Peptidbindungsknüpfung mittels Mischanhydridmethode mit Pivalinsächlorechlorid als Aktivierungsreagenz

10mmol N-terminal geschützte Aminosäure bzw. Peptid werden in 20ml absolutem THF gelöst. Die Lösung wird auf 0°C abgekühlt. Nacheinander werden unter Rühren je 10mmol N-MM und 10mmol Pivalinsäurechlorid zugegeben, wobei streng auf die Einhaltung des angegebenen Temperaturbereichs geachtet wird. Nach ca 6min wird der Ansatz auf -15°C abgekühlt und es werden nach Erreichen der tieferen Temperatur 10mmol der Aminokomponente zugesetzt. Handelt es sich bei der Aminokomponente um ein Salz, wird der Reaktionsansatz anschließend mit weiteren 10mmol N-MM versetzt. Danach wird die Reaktionsmischung 2h in der Kälte und über Nacht bei RT gerührt.

### Die weitere Aufarbeitung erfolgt wie bei Methode A.

### Methode C: Peptidbindungsknüpfung mit TB TU als Aktivierungsreagenz

10mmol der N-terminal geschützten Aminosäure bzw. Peptid und 10mmol der C-terminal geschützten der Aminokomponente werden in 20ml absolutem DMF gelöst. Die Lösung wird auf 0°C abgekühlt. Nacheinander werden unter Rühren je 10mmol DIPEA und 10mmol TBTU zugegeben. Der Ansatz wird ein Stunde bei 0°C und anschließend über Nacht bei RT gerührt. Das DMF wird vollständig i. Vak. entfernt und das Produkt wie *unter Methode A* beschrieben aufgearbeitet.

### Methode D: Darstellung eines Aktivesters (N-Hydroxysuccinimid-Ester)

10mmol N-terminal geschützte Aminosäure bzw. Peptid und 10mmol N-Hydroxysuccinimid werden in 20ml absolutem THF gelöst. Die Lösung wird auf 0°C abgekühlt, und es werden 10mmol Diyclohexylcarbodiimid unter Rühren zugegeben. Der Reaktionsansatz wird weitere 2h bei 0°C und anschließend bei RT über Nacht gerührt. Der entstandene N,N'-Dicyclohexylharnstoff wird abfiltriert, das LM i. Vak entfernt und das zurückbleibende Produkt aus EE/Pentan umkristallisiert.

### Methode E: Amidbindungsknüpfung mit N-Hydroxysuccinimid-Estern

10mmol der C-terminal ungeschützten Aminokomponente werden in einer NaHCO₃-Lösung (20mmol in 20ml Wasser) vorgelegt. Zu dieser Lösung werden bei RT unter Rühren langsam 10mmol des in 10ml Dioxan gelösten N-terminal geschützten N-Hydroxysuccinimidesters zugetropft. Der Reaktionsansatz wird weiter über Nacht gerührt und anschließend das Lösungsmittel

### i. Vak. entfernt.

### Die weitere Aufarbeitung erfolgt wie bei Methode A

### Methode F: Abspaltung der Boc-Schutzgruppe

1mmol Boc-geschütztes Aminosäurepyrrolidid, -thiazolidid oder Peptid wird mit 3ml 1,1n HCl/Eisessig (Methode *F1*) oder 3ml 1,1n HCl/Dioxan *(Methode F2)* oder 3ml 50%ige TFA in DCM *(Methode F3)* versetzt. Die Abspaltung bei RT wird mittels DC verfolgt. Nach Beendigung der Reaktion (ca 2h) wird die Verbindung als Hydrochlorid mit absolutem Diethylether ausgefällt, abgesaugt und i. Vak. über P₄O₁₀ getrocknet. Das Produkt wird aus Methanol/Ether umkristallisiert oder umgefällt.

### Methode G: Verseifung

1mmol Peptidmethylester wird in 10ml Aceton und 1ml 0,1M NaOH-Lösung gelöst und bei RT gerührt. Der Verlauf der Verseifung wird mittels DC verfolgt. Nach Beendigung der Reaktion wird das Aceton i. Vak. entfernt. Die verbleibende wässrige Lösung wird mit konzentrierter KH₂SO₄-Lösung bis zum Erreichen eines pH-Wertes von 2-3 angesäuert. Anschließend extrahiert man das Produkt mehrmals mit EE und wäscht die vereinigten Essigesterfraktionen mit gesättigter Nacl-Lösung, trocknet über NaSO₄ und entfernt i. Vak. das Lösungsmittel. Die Kristallisation erfolgt aus EE/Pentan.

### Abbildung 1:

Dargestellt ist der zeitliche Verlauf der prozentualen Hemmung der Plasma-DP IV-Aktivität nach oraler Applikation von 5µMol Inhibitor pro 300g Kalte (n=2)

### Literaturverzeichnis

Ansorge, S., Schön, E., and Kunz, D. (1991). Membrane-bound peptidases of lymphocytes: functional implications. *Biomed.Biochim.Acta* **50,** 799-807.
Ansorge, S., Bühling, F., Hoffmann, T., Kähne, T., Neubert, K., and Reinhold, D. (1995). *Dipeptidylpeptidase IV (CD26) in metabolism and the immune response* (Fleischer, B., Ed.). Springer-Verlag, Heidelberg. 163-184.
Augustyns, K., Bal, G., Thonus, G., Belyaev, A., Zhang, X. M., Bollaert, W., Lambeir, A. M., Durinx, C., Goossens, F., and Haemers, A. (1999). The unique properties of dipeptidylpeptidase IV (DPP IV/CD26) and the therapeutic potential of DPP IV inhibitors. *Curr Medicina Chem* **6**,311-327.
Buntrock, P., Neubert, K., Koh1, A., Moch, C., Born, I., Demuth, H.-U., and Barth, A. (1988). Stimulation and inhibition of wound healing process using short-chain peptides. *Biol.Zent.bl.* **107**, 87-92.
Demuth, H.-U., Heins, J., On the catalytic Mechanism of Dipeptidyl Peptidase IV. in *Dipeptidyl Peptidase IV,* Fleischer, Ed., R.G.Landes, Biomedical Publishers, Georgetown, 1, 1995.
Demuth, H.-U., McIntosh, C. H. S., Rosche, F., Pauly, R. P., Pederson, R. A., and Schmidt, J. Verfahren zur Senkung des Blutglukosespiegels in Säugern. DE 196 16 486.9
Fujita, K., Hagihara, M., Nagatsu, T., Iwata, H., and Miura, T. (1992). The activity of dipeptidyl peptidase II and dipeptidyl peptidase IV in mice immunized with type II collagen. *Biochem.Med.Metab.Biol.* **48**, 227-234.
Gomez, S., Gluschankof, P., Lepage, A., and Cohen, P. (1988). Relationship between endo-and exopeptidases in a processing enzyme system: activation of an endoprotease by the aminopeptidase B-like activity in somatostatin-28 convertase. *Proc Natl Acad Sci USA* **85**, 5468-5472.
Hegen, M., Niedobitek, G., Klein, C.E., Stein, H., and Fleischer, B. (1990). The T cell triggering molecule Tp103 is associated with dipeptidyl aminopeptidase IV activity. *J. Immunol.* **144**,2908-2914.
Hegen, M., Mittrücker, H.-W., Hug, R., Demuth, H.U., Neubert, K., Barth, A., and Fleischer, B. (1993). Enzymatic activity of CD26 (dipeptidylpeptidase IV) is not required for its signalling function in T cells. *Immunobiol.* **189**, 483-493.
Ishiura, S., Tsukahara, T., Tabira, T., and Sugita, H. (1989). Putative N-terminal splitting enzyme of amyloid A4 peptides is the multicatalytic proteinase, ingensin, which is widely distributed in mammalian cells. *FEBS Lett* **257**, 388-392.
Kameoka, J., Sato, T., Torimoto, Y., Sugita, K., Soiffer, RJ., Schlossman, S.F., Ritz, J., and Morimoto, C. (1995). Differential CD26-mediated activation of the CD3 and CD2 pathways after CD6-depleted allogeneic bone marrow transplantation. *Blood* **85**, 1132-1137.
Kähne, T., Neubert, K., and Ansorge, S. (1995). Enzymatic activity of DPIV / CD26 is involved in PMA-induced hyperphosphorylation of p56^{lck}. *Immunol. Lett.* **46**, 189-193.
Kaspari, A., Diefenthal, T., Grosche, G., Schierhorn, A., Demuth, H.-U., Substrates containing phosphorylated residues adjacent to proline decrease cleavage by proline-specific peptidases, Protein Struct Mol Enzym, 147, 1996
Kessler, H. (1982). Konformation und biologische Wirkung von zyklischen Peptiden. *Angew. Chem.* **94**, 509
Kirschke, H., Barrett, A.J., and Rawlings, N.D. (1995). Proteinases 1: lysosomal cysteine proteinases. *Protein Profile* **2**, 1581-1643.
Kohl, A., Volk, H.D., Diezel, W., Buntrock, P., and Diamantstein, T. (1989). The dipeptide Lys-Pro restores the diminished wound healing following treatment with anti-T-helper cell monoclonal antibody. *Int.J.Immunopharmacol.* **11,** 237-240.
Kohl, A., Volk, H.D., Buntrock, P., Kohl, G., Diamantstein, T., and von Baehr, R. (1991). The role of dipeptidylpeptidase IV positive T cells in wound healing and angiogenesis. *Agents Actions* **32**, 125-127.
Kräusslich, H.-G. and Wimmer, E. (1987). Viral Proteinases. *Ann. Rev. Biochem.* **57**, 701
Kubota, T., Flentke, G.R., Bachovchin, W.W., and Stollar, B.D. (1992). Involvement of dipeptidyl peptidase IV in an in vivo immune response. *Clin. Exp. Immunol.* **89**, 192-197.
Küllertz, G. and Boigk, J. (1986). Dipeptidyl peptidase IV activity in the serum and synovia of patients with rheumatoid arthritis. *Z Rheumatol* **45**, 52-56.
Levy, M.T., McCaughan, G.W., Abbott, C.A., Park, J.E., Cunningham, A.M., Müller, E., Rettig, WJ., Gorell, M.D., Fibroblast Activation Protein: A Cell Surface Dipeptidyl Peptidase and Gelatinase Expressed by Stellate Cells at the Tissue Remodelling Interface in Human Cirrhosis *Hepatology,* **29**, 1999, 1768-1778.
Nelson, PJ. and Krensky, A.M. (1998). Chemokines, lymphocytes and viruses: what goes around, comes around. *Curr Opin Immunol* **10,** 265-270.
Niedermeyer, J., Enenkel, B., Park, J. E., Lenter, M., Rettig, W. J., Damm, K., and Schnapp, A. (1998). Mouse fibroblast-activation protein - Conserved Fap gene organization and biochemical function as a serine protease. *Eur J Biochem* **254**, 650-654.
Novelli, M., Savoia, P., Fierro, M. T., Verrone, A., Quaglino, P., and Bernengo, M.G. (1996). Keratinocytes express dipeptidyl-peptidase IV (CD26) in benign and malignant skin diseases. *Br J Dermatol* **134**,1052-1056.
Pauly, R. P., Demuth, H. U., Rosche, F., Schmidt, J., White, H. A., Lynn, F., McIntosh, C. H. S., and Pederson, R. A. (1999). Improved glucose tolerance in rats treated with the dipeptidyl peptidase IV (CD26) inhibitor ile-thiazolidide. *Metabolism* **48**, 385-389.
Pauly, R. P., Rosche, F., Wermann, M., McIntosh, C. H. S., Pederson, R. A., and Demuth, H. U. (1996). Investigation of glucose-dependent insulinotropic polypeptide-(1- 42) and glucagon-like peptide-1-(7-36) degradation in vitro by dipeptidyl peptidase IV using matrixassisted laser desorption/ionization time of flight mass spectrometry - A novel kinetic approach. *J Biol Chem* **271**, 23222-23229.
Proost, P., De Meester, I., Schols, D., Struyf, S., Lambeir, A.M., Wuyts, A., Opdenakker, G., De Clercq, E., Scharp□S., and Van Damme, J. (1998a). Amino-terminal truncation of chemokines by CD26/dipeptidyl-peptidase IV. Conversion of RANTES into a potent inhibitor of monocyte chemotaxis and HIV-1-infection. *J Biol Chem* **273**, 7222-7227.
Proost, P., Struyf, S., Schols, D., Durinx, C., Wuyts, A., Lenaerts, J.P., De Clercq, E., De Meester, I., and Van Damme, J. (1998b). Processing by CD26/dipeptidyl-peptidase IV reduces the chemotactic and anti-HIV-1 activity of stromal-cell-derived factor-1alpha. *FEBS Lett* **432**, 73-76.
Proost, P., Struyf, S., Schols, D., Opdenakker, G., Sozzani, S., Allavena, P., Mantovani, A., Augustyns, K., Bal, G., Haemers, A., Lambeir, A.M., Scharp□S., Van Damme, J., and De Meester, I. (1999). Truncation of macrophage-derived chemokine by CD26/ dipeptidylpeptidase IV beyond its predicted cleavage site affects chemotactic activity and CC chemokine receptor 4 interaction. *J Biol Chem* **274**, 3988-3993.
Raynaud, F., Bauvois, B., Gerbaud, P., and Evain Brion, D. (1992). Characterization of specific proteases associated with the surface of human skin fibroblasts, and their modulation in pathology. *J.Cell.Physiol.* **151**, 378-385.
Reinhold, D., Bank, U., Buhling, F., Tager, M., Born, I., Faust, J., Neubert, K., and Ansorge, S. (1997). Inhibitors of dipeptidyl peptidase IV (DP IV, CD26) induces secretion of transforming growth factor-beta 1 (TGF-beta 1) in stimulated mouse splenocytes and thymocytes. *Immunol Lett* **58**, 29-35.
Reinhold, D., Hemmer, B., Gran, B., Born, I, Faust, J., Neubert, K., McFarland, H. F., Martin, R., and Ansorge, S. (1998). Inhibitors of dipeptidyl peptidase IV/CD26 suppress activation of human MBP-specific CD4+T cell clones. *J Neuroimmunol* **87**, 203-209.
Reinhold D., Hemmer, B., Steinbrecher, A., Born, I., Faust, J., Neubert, K., Brocke, S., Martin, R., Ansorge, S., Dipeptidyl Peptidase IV (CD 26): Role in T cell activation and autoimmune disease. Biol. Chem.379, S 69, 1998.
Scholzen, T., Armstrong, C.A., Bunnett, N.W., Luger, T.A., Olerud, J.E., and Ansel, J.C. (1998). Neuropeptides in the skin: interactions between the neuroendocrine and the skin immune systems. *Exp Dermatol* **7***,* 81-96.
Shioda, T., Kato, H., Ohnishi, Y., Tashiro, K., Ikegawa, M., Nakayama, E.E., Hu, H., Kato, A., Sakai, Y., Liu, H., Honjo, T., Nomoto, A., Iwamoto, A., Morimoto, C., and Nagai, Y. (1998). Anti-HIV-1 and chemotactic activities of human stromal cell-derived factor 1alpha (SDF-1alpha) and SDF-1beta are abolished by CD26/dipeptidyl peptidase IV-mediated cleavage. *Proc Natl Acad Sci USA* **95**, 6331-6336.
Tanaka, T., Kameoka, J., Yaron, A., Schlossman, S.F., and Morimoto, C. (1993). The costimulatory activity of the CD26 antigen requires dipeptidyl peptidase IV enzymatic activity. *Proc.NatlAcad.Sci.U.S.A.* **90**, 4586-4590.
Tanaka, S., Murakami, T., Horikawa, H., Sugiura, M., Kawashima, K., and Sugita, T. (1997). Suppression of arthritis by the inhibitors of dipeptidyl peptidase IV. *Int J Immunopharmacol* **19,** 15-24.
Tanaka, S., Murakami, T., Nonaka, N., Ohnuki, T., Yamada, M., and Sugita, T. (1998). Antiarthritic effects of the novel dipeptidyl peptidase IV inhibitors TMC-2A and TSL-225. *Immunopharmacology* **40,** 21-26.
Vanhoof, G., Goossens, F., De Meester I, Hendriks, D., and Scharpe, S. (1995). Proline motifs in peptides and their biological processing. *FASEB J*. **9,** 736-744.
Wallengren, J. (1997). Vasoactive peptides in the skin. *J Investig Dermatol Symp Proc* **2,** 49-55.
Yaron, A. and Naider, F. (1993). Proline-dependent structural and biological properties of peptides and proteins. *Crit.Rev.Biochem.Mol.Biol.* **28(1)**, 31-38.

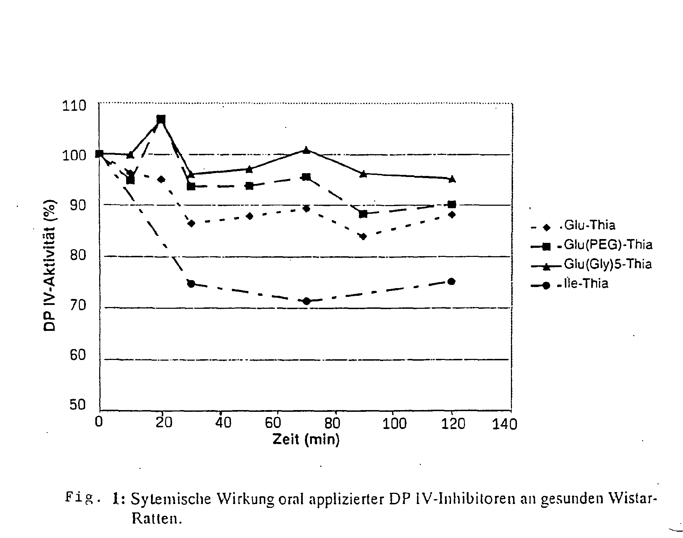

## Patentansprüche

1. Verbindung der allgemeinen Formel 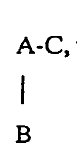 wobei
A eine Aminosäure mit mindestens einer funktionellen Gruppe in der Seitenkette ist,
B eine chemische Verbindung ist, die kovalent an mindestens eine funktionelle Gruppe der Seitenkette von A gebunden ist, nämlich
- Oligopeptide mit einer Kettenlänge von bis zu 20 Aminosäuren außer Homopolymeren von Glycin aus bis zu 6 Glycinmonomeren oder
- Polyethylenglykole mit Molmassen von bis zu 20000 g/mol,
C eine Thiazolidin-, Pyrrolidin-, Cyanopyrrolidin-, Hydroxyprolin-, Dehydroprolin- oder Piperidingruppe ist, die in Amidbindung mit A vorliegt.

2. Verbindung nach Anspruch 1, **dadurch gekennzeichnet, daß** A eine α-Aminosaure ist.

3. Verbindung nach einem der vorstehenden Ansprüche, **dadurch gekennzeichnet, daß** A eine natürliche α-Aminosäure ist.

4. Verbindung nach einem der vorstehenden Ansprüche, **dadurch gekennzeichnet, daß** die Aminosäure Threonin, Tyrosin, Serin, Arginin, Lysin, Asparaginsäure, Glutaminsäure oder Cystein ist.

5. Verbindung nach einem der vorstehenden Ansprüche, **dadurch gekennzeichnet, daß** die Oligopeptide Kettenlängen von 3 bis 15 Aminosäuren aufweisen.

6. Verbindung nach einem der vorstehenden Ansprüche, **dadurch gekennzeichnet, daß** die Oligopeptide Homopolymere, Copolymere oder Blockcopolymere sind.

7. Verbindung nach einem der vorstehenden Ansprüche, **dadurch gekennzeichnet, daß** die Polyethylenglykole Molmassen von mindestens 250 g/mol aufweisen.

8. Verbindung nach einem der vorstehenden Ansprüche, **dadurch gekennzeichnet, daß** C eine Thiazolidin- Pyrrolidin-, oder Cyanopyrrolidingruppe ist.

9. Pharmazeutische Zusammensetzung, die eine Verbindung nach einem der vorstehenden Ansprüche gegebenenfalls in Kombination mit an sich üblichen Trägern oder Adjuvantien enthält.

10. Kosmetische Zusammensetzung, die eine Verbindung nach einem der Ansprüche 1 bis 8 gegebenenfalls in Kombination mit an sich üblichen Trägern oder Adjuvantien enthält.

11. Verwendung von mindestens einer Verbindung nach einem der Ansprüche 1 bis 8 zur Herstellung eines Arzneimittels zur topischen Beeinflussung der Aktivität von Dipeptidyl-Peptidase IV oder analoger Enzyme.

12. Verwendung von mindestens einer Verbindung nach einem der Ansprüche 1 bis 8 zur Herstellung eines Arzneimittels zur Prophylaxe oder Therapie von Haut- oder Schleimhauterkrankungen, Autoimmunerkrankungen und Entzündungen.

13. Verwendung von mindestens einer Verbindung nach einem der Ansprüche 1 bis 8 zur Herstellung eines Arzneimittels zur Prophylaxe oder Therapie von Entzündungen, Psoriasis, Allergien, Arthritis, Tumoren oder Autoimmunerkrankungen.

14. Verwendung von mindestens einer Verbindung oder pharmazeutischen oder kosmetischen Zusammensetzung nach einem der Ansprüche 1 bis 10 in Salbe, Creme, Kosmetikum, Pflaster, Binde, Tropfen, Spray, Inhalat, Implantat oder Injektionslösung.

## Claims

1. Compound of the general formula 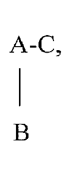 wherein
A is an amino acid having at least one functional group in the side chain,
B is a chemical compound covalently bound to at least one functional group of the side chain of A, namely
- oligopeptides having a chain length of up to 20 amino acids, except for homopolymers of glycine consisting of up to 6 glycine monomers, or
- polyethylene glycols having molar masses of up to 20 000 g/mol,
C is a thiazolidine, pyrrolidine, cyanopyrrolidine, hydroxyproline, dehydroproline or piperidine group amide-bonded to A.

2. Compound according to claim 1, **characterised in that** A is an α-amino acid.

3. Compound according to one of the preceding claims, **characterised in that** A is a natural α-amino acid.

4. Compound according to one of the preceding claims, **characterised in that** the amino acid is threonine, tyrosine, serine, arginine, lysine, asparagine, glutamic acid or cysteine.

5. Compound according to one of the preceding claims, **characterised in that** the oligopeptides have chain lengths of from 3 to 15 amino acids.

6. Compound according to one of the preceding claims, **characterised in that** the oligopeptides are homopolymers, copolymers or block copolymers.

7. Compound according to one of the preceding claims, **characterised in that** the polyethylen glycols have molar masses of at least 250 g/mol.

8. Compound according to one of the preceding claims, **characterised in that** C is a thiazolidine, pyrrolidine or cyanopyrrolidine group.

9. Pharmaceutical composition that comprises a compound according to one of the preceding claims, optionally in combination with carriers or adjuvants customary *per se.*

10. Cosmetic composition that comprises a compound according to one of claims 1 to 8, optionally in combination with carriers or adjuvants customary per se.

11. Use of at least one compound according to one of claims 1 to 8 for the preparation of a medicament for topically influencing the activity of dipeptidyl peptidase IV or of analogous enzymes.

12. Use of at least one compound according to one of claims 1 to 8 for the preparation of a medicament for prophylaxis or therapy of diseases of the skin or mucosa, autoimmune diseases and inflammation.

13. Use of at least one compound according to one of claims 1 to 8 for the preparation of a medicament for prophylaxis or therapy of inflammation, psoriasis, allergies, arthritis, tumours or autoimmune diseases.

14. Use of at least one compound or pharmaceutical or cosmetic composition according to one of claims 1 to 10 in an ointment, a cream, a cosmetic, a patch, a dressing, drops, a spray, an inhalation, an implant, or an injection solution.

## Revendications

1. Composé de formule générale 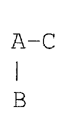 où
A est un acide aminé ayant au moins un groupe fonctionnel dans la chaîne latérale,
B est un composé chimique, qui est lié de manière covalente à au moins un groupe fonctionnel de la chaîne latérale de A, notamment
- un oligopeptide ayant une longueur de chaîne allant jusqu'à 20 acides aminés hormis les homopolymères de glycine ayant jusqu'à 6 monomères de glycine ou
- un polyéthylène glycol, ayant des masses molaires allant jusqu'à 20000 g/mol,
C est un groupe thiazolidine, pyrrolidine, cyanopyrrolidine, hydroxyproline, déhydroproline ou pipéridine, qui se présente en liaison amide avec A.

2. Composé selon la revendication 1, **caractérisé en ce que** A est un acide α-aminé.

3. Composé selon l'une des revendications précédentes, **caractérisé en ce que** A est un acide a-aminé naturel.

4. Composé selon l'une des revendications précédentes, **caractérisé en ce que** l'acide aminé est la thréonine, la tyrosine, la sérine, l'arginine, la lysine, l'acide asparaginique, l'acide glutamique ou la cystéine.

5. Composé selon l'une des revendications précédentes, **caractérisé en ce que** les oligopeptides présentent des longueurs de chaîne de 3 à 15 acides aminés.

6. Composé selon l'une des revendications précédentes, **caractérisé en ce que** les oligopeptides sont des homopolymères, copolymères ou copolymères blocs.

7. Composé selon l'une des revendications précédentes, **caractérisé en ce que** les polyéthylène glycols présentent des masses molaires d'au moins 250 g/mol.

8. Composé selon l'une des revendications précédentes, **caractérisé en ce que** C est un groupe thiazolidine, pyrrolidine ou cyanopyrrolidine.

9. Composition pharmaceutique, qui contient un composé selon l'une des revendications précédentes, le cas échéant en combinaison avec des supports ou adjuvants usuels en soi.

10. Composition cosmétique, qui contient un composé selon l'une des revendications 1 à 8, le cas échéant en combinaison avec des supports ou adjuvants usuels en soi.

11. Utilisation d'au moins un composé selon l'une des revendications 1 à 8 pour la préparation d'un médicament destiné à influencer topiquement l'activité de la dipeptidyl-peptidase IV ou des enzymes analogues.

12. Utilisation d'au moins un composé selon l'une des revendications 1 à 8 pour la préparation d'un médicament destiné à la prophylaxie ou la thérapie des maladies de la peau ou des muqueuses, des maladies autoimmunes et des inflammations.

13. Utilisation d'au moins un composé selon l'une des revendications 1 à 8 pour la préparation d'un médicament destiné à la prophylaxie ou la thérapie des inflammations, du psoriasis, des allergies, de l'arthrite, des tumeurs ou des maladies autoimmunes.

14. Utilisation d'au moins un composé ou une composition pharmaceutique ou cosmétique selon l'une des revendications 1 à 10 dans une pommade, une crème, un produit cosmétique, un pansement, une bande, des gouttes, un spray, un produit à inhaler, un implant ou une solution à injecter.
